(19) Europäisches Patentamt
European Patent Office
Office européen des brevets

(11) Veröffentlichungsnummer: **0 180 108**
**B1**

(12) # EUROPÄISCHE PATENTSCHRIFT

(45) Veröffentlichungstag der Patentschrift: **02.08.89**

(51) Int. Cl.[4]: **C 07 C 29/17**, C 07 C 33/025, C 07 C 33/03, C 07 C 33/02, C 07 C 67/283, C 07 C 69/145, C 07 C 41/20

(21) Anmeldenummer: **85113223.3**

(22) Anmeldetag: **18.10.85**

(54) **Verfahren zur Hestellung von olefinisch ungesättigten Verbindungen, insbesondere Alkenolen.**

(30) Priorität: **24.10.84 DE 3438851**

(43) Veröffentlichungstag der Anmeldung: **07.05.86 Patentblatt 86/19**

(45) Bekanntmachung des Hinweises auf die Patenterteilung: **02.08.89 Patentblatt 89/31**

(84) Benannte Vertragsstaaten: **AT BE CH DE FR GB IT LI NL**

(56) Entgegenhaltungen:
**EP-A-0 011 439**
**EP-A-0 032 396**

(73) Patentinhaber: **BASF Aktiengesellschaft, Carl-Bosch- Strasse 38, D-6700 Ludwigshafen (DE)**

(72) Erfinder: **Seufert, Walter, Dr., Laerchenweg 19, D-6720 Speyer (DE)**
Erfinder: **Goetz, Norbert, Dr., Schoefferstrasse 25, D-6520 Worms 1 (DE)**
Erfinder: **Theobald, Hans, Dr., Queichstrasse 6, D-6703 Limburgerhof (DE)**
Erfinder: **Schwendemann, Volker, Dr., 35 Oak Lane, Mountain Lakes, N.J. 07046 (US)**

**EP 0 180 108 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft ein Verfahren zur Herstellung von olefinisch ungesättigten Verbindungen durch Hydrierung von entsprechenden acetylenisch ungesättigten Verbindungen an einem Palladiumkatalysator. Sie betrifft insbesondere die Herstellung der cis-Isomeren von Alkenylverbindungen der Formel I

$$CH_3(CH_2)_m\text{-}CH{=}CH\text{-}(CH_2)_n\text{-}OR \quad (I)$$

in einer Reinheit, wie sie bisher nicht erzielt wurde, durch partielle Hydrierung der entsprechenden Alkinylverbindungen an einem vorhydrierten Trägerkatalysator, der Palladium enthält.

Für viele, insbesondere biologische Zwecke, z. B. bei Geruchsstoffen, Wachstumsregulatoren, Pharmaka, kommt es auf die Reinheit, und zwar, wo dies strukturell gegeben ist, auch auf die sterische Einheitlichkeit der eingesetzten Verbindungen an. Beispielsweise ist die Reinheit synthetischer Pheromone im allgemeinen von großer Bedeutung. Eine potentielle Inhibitorwirkung von Positions- oder Stereoisomeren erfordert Synthesemethoden, die von vorn herein Stoffe hoher chemischer und stereochemischer Reinheit liefern, da eine nachträgliche Abtrennung oft aussichtslos ist.

An sich ist bekannt, daß die Anlagerung von Wasserstoff an die C=C-Dreifachbindung unter Bildung der cis-Olefine verlaufen kann (vgl. Houben-Weyl, Methoden der Organischen Chemie, 4. Aufl., Band 4/1c, Seiten 76 ff und 110). Dabei erweist sich der sogenannte Lindlar-Katalysator (Palladium-Katalysator auf Kalziumcarbonat-Basis mit Bleiacetat-Zusatz) in der Regel in der sterischen Einheitlichkeit der Hydrierprodukte anderen Katalysatoren überlegen. Aber auch mit den Lindlar-Katalysatoren entstehen neben dem erwünschten cis-Isomeren auch z. B. 4 und mehr Prozent des Trans-Isomeren.

Ein besonderes Problem stellt die selektive cis-Hydrierung von Alkinolen zu den erfindungsgemäßen Alkenolen (I) dar. Beschrieben ist die Hydrierung bei 65 % Umsatz und einem nicht exakt bestimmten Anteil an E-Isomeren (nur NMR- und IR-Analytik) von J.K. Crandall et al., J. Org. Chem. 41, 4089 (1976).

Eine von Disselnkötter et al., Tetrahedron 1976, 1591 und Rossi et al., Gazetta Chimica Italiana 108, 709 (1978) publizierte Methode zur Hydrierung von Octin-3-ol-1 zu Octen-3-ol-1 mit modifiziertem Lindlar-Katalysator (5 g 1 % Pd/$CaCO_3$, mit 20 mg Bleiacetat-Zusatz) ohne Angabe des E-Isomerenanteils haben wir nachgearbeitet und erhielten 96 % cis-Octen-3-ol-1, 2,9 % trans-Octen-3-ol-1, 0,6 % Octanol-1. Hierzu wurden 5,7 Gew.-% $PdO_2$ und 7,5 Gew.% Pb $(OAc)_2$ auf $CaCO_3$ aufgebracht und in üblicher Weise reduziert: 20 g des Katalysators wurden in 2 l Methanol suspendiert, vorhydriert und bei Raumtemperatur 443 g Octin-3-ol-1 hydriert. Wiedergewonnen wurden 338 g Material, das die vorstehende Zusammensetzung hatte.

Die höchsten Zahlenwerte der sterischen Einheitlichkeit scheinen bei der in EP-PS-11 439 beschriebenen Methode erzielbar; jedoch ist in keinem der Beispiele ein cis-Isomeren-Anteil von mehr als 98,2 % oder trans-Isomeren-Anteil von weniger als 1,6 % angegeben. Außerdem erfordern die vorgeschlagenen Hydrierungen mindestens 5 bar Wasserstoffdruck im Autoklaven also den Einsatz teurer Spezialapparaturen. Die Angaben der EP-PS reichten für eine Nachprüfung nicht aus.

Es bestand daher ein Bedürfnis nach einem technisch einsetzbaren Verfahren, das es erlaubt, acetylenisch ungesättigte Verbindungen, insbesondere Alkin-2-ole-1 und Alkin-3-ole-1 bzw. deren Derivate möglichst vollständig und nebenproduktarm zu den cis-Alken-2-olen-1 und cis-Alken-3-olen-1 zu hydrieren. Die acetylenisch ungesättigten Verbindungen sind oft besonders leicht und in guter Reinheit erhältlich.

Die Lösung der Aufgabe besteht in dem Verfahren nach den Patentansprüchen. Wichtig ist dabei eine sorgfältige erschöpfende Hydrierung des Katalysators vor dessen Einsatz. Nach dieser Vorhydrierung sind Anteile an cis-Isomeren von bis zu 99,9 % zu erhalten. Es ist überraschend, daß ein mit Silber abgeschwächter Palladiumkatalysator bei milden Bedingungen sehr selektiv Dreifachbindungen zu olefinischen Doppelbindungen zu hydrieren vermag, denn mit den schon zu anderen Zwecken verwendeten Silber-Palladium-Katalysatoren wurden gerade olefinisch ungesättigte Verbindungen zu aliphatischen Verbindungen hydriert (vgl. DE-OS-1 518 598).

Der erfindungsgemäß zu verwendende Katalysator enthält Palladium und Silber, vorzugsweise auf einem Aluminiumoxidträger. Als Träger eignen sich ggf. auch Kieselgel, Aluminiumsilikat, Spinelle des Aluminiums oder Kalziumcarbonat. Der Palladium-Gehalt, bezogen auf den Katalysator einschießlich Trägermaterial, liegt im Bereich von 0,05 bis 15, vorzugsweise 0,1 bis 5 Gew.-%, berechnet als Metall, der des Silbers 0,5 bis 15, vorzugsweise 1 bis 10 Gew.%. Das Gewichtsverhältnis von Silber zu Palladium kann unterschiedlich sein, z. B. im Bereich von 300 : 1 bis 1 : 30. Günstig sind Katalysatoren, die relativ wenig Palladium (z. B. 0,1 bis 2 %) und eine etwas höhere Menge Silber enthalten. Die Herstellung von Silber-Palladium-Katalysatoren ist z. B. in den DE-PSen-1 518 598 und 1 179 947 beschrieben, allerdings wird bei diesen Katalysatoren stets Kieselsäure oder ein Silikat als Träger verwendet.

Man verwendet den Katalysator meist in Form von Strängen (beim Hydrieren im Festbett) oder als Pulver (beim Arbeiten in Suspension).

Geeignete Lösungsmittel sind vor allem Alkohole, insbesondere $C_1$- bis $C_4$-Alkohole.

Die Reaktion verläuft schon oberhalb von etwa -10°C, befriedigend rasch, vorzugsweise zwischen 10 und 50°C. Der erforderliche Wasserstoffmindestdruck beträgt in der Regel von atmosphärischem Druck bis etwa 3 bar, vorzugsweise bis 1,5 bar; ein Druck von 5 bar oder mehr ist im allgemeinen unwirtschaftlich und erfordert besondere Apparate, da die Umsetzung dann sehr rasch verläuft; u.U. tritt bei zu hohem Druck auch der gewünschte Erfolg nicht ein. Weiterhin sollte darauf geachtet werden, daß die insgesamt anwesende

Palladiummenge möglichst gering ist; wenn also ein Katalysator z. B. 0,5 % Palladium enthält, so liefert eine 1 gew.-%-ige Katalysatorsuspension gute Ergebnisse; das gleiche gilt für eine 0,2 %-ige Suspension eines 2,5 Gew.-% Pd enthaltenden Katalysators; setzt man von diesem Katalysator jedoch eine 1 %-ige Suspension an, so kann der Gehalt an cis-Isomeren geringer sein als erwartet.

R in Formel I ist vorzugsweise Wasserstoff. Als leicht abspaltbare Schutzgruppe, die den Wasserstoff ersetzen kann, kommen beispielsweise in Betracht: Tetrahydropyranyl-2, Trialkylsilyl, Acetyl, tert.-Butyl und Benzyl. Natürlich können auch Verbindungen in die cis-Olefinstruktur überführt werden, bei denen R keine Schutzgruppe bedeutet, sondern einen Bestandteil des Endprodukts, wie z. B. Ester der Hydroxylverbindungen mit gesättigten oder ungesättigten Fettsäuren oder Ether, vorzugsweise Alkylether.

**Beispiel 1**

10 g Palladiumoxid-Silberoxid-Katalysator (0,5 % Palladium + 5 % Silber auf Aluminiumoxid) werden in 1500 ml Methanol suspendiert und 30 Minuten bei 20°C mit Wasserstoff behandelt. Dann werden 156 g Octin-3-ol-1 zugesetzt und bei 20 bis 30°C unter einem Druck von 1,1 bar bis zum Ende der Wasserstoffaufnahme hydriert (ca. 3 Stunden).

Nach Abziehen des Methanols und Destillation bei 47°C/0,1 bar erhält man reines cis-Octen-3-ol-1. Das trans-Isomere ist gaschromatographisch und NMR-spektroskopisch nicht nachweisbar.

Nach dem Vorbild des Beispiels 1 wurden hergestellt:

| Beispiel/Alkenol | cis-Anteil | trans-Anteil |
|---|---|---|
| 2 Hepten-3-ol-1 | 99,0 % | 0,3 % |
| 3 Decen-3-ol-1 | 98,2 % | 0,4 % |
| 4 Dodecen-3-ol-1 | 98,0 % | 0,4 % |
| 5 Tetradecen-3-ol-1 | 96,7 % | 0,5 % |
| 6 Penten-2-ol-1 | 97,6 % | 0,3 % |
| 7 Hexen-2-ol-1 | 99,0 % | 0,4 % |
| 8 Hexen-3-ol-1 | 98,8 % | 0,6 % |
| 9 Hepten-2-ol-1 | 97,6 % | 0,5 % |
| 10 Nonen-2-ol-1 | 99,0 % | 0,2 % |
| 11 Undecen-2-ol-1 | 98,9 % | 0,4 % |

Der Rest zu 100 % sind Lösungsmittelspuren sowie die jeweilige gesättigte Verbindung.

Nach dieser Vorschrift können beispielsweise auch hergestellt werden:

Penten-3-ol-1
Nonen-3-ol-1
Undecen-3-ol-1
Buten-2-ol-1
Octen-2-ol-1
Decen-2-ol-1
Dodecen-2-ol-1

sowie die entsprechenden Acetate, tert.-Butyl-, Benzyl-, 2-Tetrahydropyranyl- und Trimethylsilylderivate (vgl. auch folgende Beispiele).

**Beispiel 12**

Durch Hydrierung von Octin-3-ol-1-acetat in einer Menge von 100 g unter den Bedingungen des Beispiels 1 wurde das entsprechende Octenolacetat hergestellt (Wasserstoffaufnahme 5 Stunden) und anschließend verseift. Bezogen auf das Ausgangsmaterial wurden 97,2 % des cis-Octenols und 1,6 % des trans-Octenols erhalten.

**Beispiel 13**

Die Beispiel 12 entsprechende TH-Pyranylverbindung wurde durch 6-stündiges Hydrieren hergestellt und verseift. Die Hydrierung lieferte praktisch reines cis-Octenol.

**Beispiel 14**

Die Beispiel 12 entsprechende Trimethylsilylverbindung lieferte innerhalb von 5 Stunden cis-Octenol in einer Ausbeute von 98,7 % und trans-Octenol in einer Ausbeute von 0,5 %.

**Beispiel 15**

Die Hydrierung von Octin-3-ol-1 zum entsprechenden Octenol wurde unter den Bedingungen des Beispiels 1 mit einem Katalysator wiederholt, der außer Palladium und Silber noch 1 Gew.-% Mangan enthielt. Das Isomerenverhältnis ergibt sich aus der Ausbeute von 95,8 % cis-Verbindung, 2,0 % trans-Verbindung und 0,8 % der gesättigten, also vollständig hydrierten Verbindung.

**Beispiel 16**

0,5 g Palladiumoxid-Silberoxid-Katalysator (0,5 % Palladium, 5,0 % Silber auf Aluminiumoxid) wurden in 500 ml Methanol suspendiert und - wie in Beispiel 1 angegeben - aktiviert. Anschließend wurden 50 g 3,7-Dimethyl-octin-1-en-6-ol-3 (Dehydrolinalool, Gehalt: 99,0 %) zugegeben und bei 20 bis 25° C und einem Druck von 1,1 bar bis zum Ende der Wasserstoffaufnahme hydriert. Es wurde filtriert, das Lösungsmittel entfernt und ein Reaktionsprodukt folgender Zusammensetzung erhalten (bestimmt mittels Kapillar-Gaschromatographie):

96,2 % 3,7-Dimethyl-octadien-1,6-ol-3 (Linalool)
2,8 % 3,7-Dimethyl-octen-6-ol-3.

**Beispiel 17**

Es wurde verfahren, wie in Beispiel 16 beschrieben, wobei ein Katalysator verwendet wurde, der 0,1 % Palladium und 5 % Silber auf Aluminiumoxid enthält.

Hierbei konnte ein Umsetzungsprodukt folgender Zusammensetzung (bestimmt mittels Kapillar-Gaschromatographie) gewonnen werden:

96,9 % 3,7-Dimethyl-octadien-1,6-ol-3 (Linalool)
2,1 % 3,7-Dimethyl-octen-6-ol-3.

**Patentansprüche**

1. Verfahren zur Herstellung von olefinisch ungesättigten Verbindungen durch Hydrierung von entsprechenden acetylenisch ungesättigten Verbindungen an einem Palladiumkatalysator, dadurch gekennzeichnet, daß man einen Trägerkatalysator verwendet, der 0,05 bis 15 Gew.-% Palladium und 0,5 bis 15 Gew.-% Silber enthält.

2. Verfahren nach Anspruch 1 zur Herstellung des cis-Isomeren einer Alkenylverbindung der Formel

$$CH_3(CH_2)_m\text{-}CH=CH\text{-}(CH_2)_n\text{-}OR \qquad (I),$$

wobei R Wasserstoff, einen Ether- oder Esterrest oder eine leichtabspaltbare Schutzgruppe bedeutet, m die Werte 1 bis 12 und n die Werte 1 oder 2 annehmen kann, durch katalytische partielle Hydrierung der entsprechenden Acetylenverbindung.

3. Verfahren nach Anspruch 1 zur Herstellung des cis-Isomeren einer Alkenylhydroxiverbindung der Formel

$$CH_3(CH_2)_m\text{-}CH=CH\text{-}(CH_2)_2OH \qquad (II).$$

4. Verfahren nach Anspruch 1 zur Herstellung von Z-3-Octenol-1.

5. Verfahren nach einem der Ansprüche 1 bis 4, dadurch gekennzeichnet, daß ein Katalysator eingesetzt wird, der Aluminiumoxid als Träger enthält.

6. Verfahren nach einem der Ansprüche 1 bis 5, dadurch gekennzeichnet, daß man den Katalysator vor der Umsetzung erschöpfend mit Wasserstoff behandelt.

7. Verfahren nach einem der Ansprüche 1 bis 6, dadurch gekennzeichnet, daß der Katalysator 1 % oder weniger an Palladium enthält.

## Claims

1. A process for the preparation of an olefinically unsaturated compound by hydrogenating the corresponding acetylenically unsaturated compound over a palladium catalyst, wherein a supported catalyst containing from 0.05 to 15 % by weight of palladium and from 0.5 to 15 % by weight of silver is used.

2. A process as claimed in claim 1 for the preparation of the cis-isomer of an alkenyl compound of the formula

$$CH_3(CH_2)_m\text{-}CH=CH\text{-}(CH_2)_n\text{-}OR \qquad (I)$$

where R is hydrogen, an ether or ester radical or a protective group which can be readily eliminated, m is from 1 to 12 and n is 1 or 2, by catalytic partial hydrogenation of the corresponding acetylene compound.

3. A process as claimed in claim 1 for the preparation of the cis-isomer of an alkenylhydroxy compound of the formula

$$CH_3(CH_2)_m\text{-}CH=CH\text{-}(CH_2)_2OH \qquad (II).$$

4. A process as claimed in claim 1 for the preparation of Z-oct-3-en-1-ol.

5. A process as claimed in any of claims 1 to 4, wherein the catalyst used contains alumina as a carrier.

6. A process as claimed in any of claims 1 to 5, wherein the catalyst is treated exhaustively with hydrogen before the reaction.

7. A process as claimed in any of claims 1 to 6, wherein the catalyst contains 1 % of palladium or less.

## Revendications

1. Procede de préparation de composés à insaturation oléfinique par hydrogénation de composés à insaturation acétylénique correspondants sur un catalyseur au palladium, caractérisé en ce qu'on utilise un catalyseur supporté qui contient de 0,05 à 15 % en poids de palladium et 0,5 à 15 % en poids d'argent.

2. Procédé selon la revendication 1, pour la préparation de l'isomère cis d'un composé alcénylique de formule

$$CH_3(CH_2)_m\text{-}CH=CH\text{-}(CH_2)_nOH \qquad (II).$$

dans laquelle R représente un atome d'hydrogène, un reste éther ou ester ou un groupement protecteur facilement dissociable, m peut prendre la valeur 1 à 12 et n la valeur 1 ou 2,

par hydrogénation partielle catalytique du composé acétylénique correspondant.

3. Procédé selon la revendication 1, pour la préparation de l'isomère cis d'un composé alcénylique hydroxylé de formule

$$CH_3(CH_2)_m\text{-}CH=CH\text{-}(CH_2)_2OH \qquad (II).$$

4. Procédé selon la revendication 1, pour la preparation du Z-3-octénol-1.

5. Procédé selon l'une quelconque des revendications 1 à 4, caractérisé en ce qu'on utilise un catalyseur qui contient de l'oxyde d'aluminium comme support.

6. Procédé selon l'une quelconque des revendications 1 à 5, caractérisé en ce qu'on traite à fond avec de l'hydrogène le catalyseur avant la réaction.

7. Procédé selon l'une quelconque des revendications 1 à 6, caractérisé en ce que le catalyseur contient 1 % ou moins de palladium.